(19) Europäisches Patentamt
European Patent Office
Office européen des brevets

(11) **EP 3 574 959 A1**

(12) **EUROPEAN PATENT APPLICATION**

(43) Date of publication:
**04.12.2019 Bulletin 2019/49**

(21) Application number: **18174893.0**

(22) Date of filing: **29.05.2018**

(51) Int Cl.:
*A62B 18/08* (2006.01)       *A61M 16/06* (2006.01)
*A62B 9/00* (2006.01)        *G01M 3/26* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **SU, Wei**
**5656 AE Eindhoven (NL)**

• **CHEN, Weizhong**
**5656 AE Eindhoven (NL)**
• **BOUMA, Peter Hermanus**
**5656 AE Eindhoven (NL)**
• **VAN DER SLUIS, Paul**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **BREATHING ASSISTANCE FACE MASK AND METHOD OF ITS CONTROL**

(57)     A fan (16) speed signal of a fan-assisted face mask (12) is processed, to provide an indication of a level of leakage to or from the mask volume (14). In this way, a wearer (10) of the mask (12) is able to ensure correct initial fitting of the mask (12), as well as to perform periodic checks of the fitting of the mask (12).

FIG. 1

## Description

FIELD OF THE INVENTION

This invention relates to breathing assistance face masks in which an electric fan draws air through an air filter into the mask or draws air out of the mask, to assist inhalation or exhalation.

BACKGROUND OF THE INVENTION

Air pollution is a worldwide concern. The World Health Organization (WHO) estimates that 4 million people die from air pollution every year. Part of this problem is the outdoor air quality in cities. Nearly 300 smog-hit cities fail to meet national air quality standards.

Official outdoor air quality standards define particle matter concentration as mass per unit volume (e.g. $\mu g/m^3$). A particular concern is pollution with particles having a diameter less than 2.5 $\mu$m (termed "PM2.5") as they are able to penetrate into the gas exchange regions of the lung (alveoli), and very small particles (<100 nm) may pass through the lungs to affect other organs.

Since this problem will not improve significantly on a short time scale, a common way to deal with this problem is to wear a mask which provides cleaner air by filtration and the market for masks in China and elsewhere has seen a great surge in recent years. For example, it is estimated that by 2019, there will be 4.2 billion masks in China.

Such masks may be made of material that acts as a filter of pollutant particles, or may have a filter for only part of the mask surface, and this filter may be replaceable when it becomes clogged.

However, during use, the temperature and relative humidity inside the mask increases and, combined with the pressure difference inside the mask relative to the outside, this makes breathing uncomfortable. This can be mitigated in part by providing an outlet valve or check valve which allows exhaled air to escape the mask with little resistance, but which requires inhaled air to be drawn through the filter. To improve comfort and effectiveness, a fan can be added to the mask, this fan drawing in air through the filter. For efficiency and longevity reasons these are normally electrically commutated brushless DC fans.

The benefit to the wearer of using a fan-powered mask is that the lungs are relieved of the slight strain caused by inhalation against the resistance of the filters in a conventional non-powered mask.

Furthermore, in a conventional non-powered mask, inhalation also causes a slight pressure drop within the mask which leads to leakage of the contaminants into the mask, which leakage could prove dangerous if these are toxic substances.

Fan-assisted masks thus may improve the wearing comfort by reducing the temperature, humidity and breathing resistance.

In one arrangement, an inlet (i.e. inhale) fan may be used to provide a continuous intake of air. In this way, the lungs are relieved of the slight strain caused by inhalation against the resistance of the filters in a conventional non-powered mask. A steady stream of air may then be provided to the face and may for example provide a slight positive pressure, to ensure that any leakage is outward rather than inward. However, this gives additional resistance to breathing when exhaling.

In another arrangement, an exhaust (i.e. exhale) fan may be used to provide a continuous release of air. This instead provides breathing assistance when exhaling, but, in contrast to the use of a passive outlet valve, this has the disadvantage that a negative pressure in the mask volume may result, so that leakage around the mask edge results in polluted air leaking into the mask volume.

Another alternative is to provide both inlet and exhaust fans, and to time the control of the fans in synchronism with the breathing cycle of the user. The breathing cycle may be measured based on pressure (or differential pressure) measurements. This provides improved control of temperature and humidity as well as reducing the resistance to breathing for both inhalation and exhalation.

By providing regulation of the fan operation in this way (and also regulation of the fan speed) more appropriate ventilation is provided during the inhalation and exhalation sequence and the electrical efficiency is improved. The latter translates into longer battery life or increased ventilation.

Thus, several types of mask for preventing daily exposure to air pollutants are available, including passive masks, passive masks with an exhale valve, and masks with at least one active fan. This invention relates in particular to masks with a fan arrangement.

One problem associated with masks is that often the wearer does not know whether the mask fits well or not. If the mask does not fit well, there will be leakage causing polluted air entering the mask. The control of the user comfort based on the fan-assisted operation will also be influenced. There are various situations which can lead to leakage problems.

If the mask is not fitted properly from the outset, then it will have leakage issues. In order to address this problem, the user instructions for the mask will instruct the user how to fit and adjust the mask when they put on the mask. However, this gives no feedback for the user.

Even if the mask is initially fitted very well, after some head activity, such as moving the head side to side, or up and down, the mask may move and introduce leakage problems.

There is a need therefore for a mask which is able to provide feedback about how well the mask fits to the user so that adjustments may be made as and when needed.

SUMMARY OF THE INVENTION

The invention is defined by the claims.

According to a first aspect of the invention, there is provided a fan-assisted face mask comprising:

a mask body which is adapted to enclose a mask volume against the face of a wearer;
a fan arrangement comprising at least one fan; and
a controller adapted to control the at least one fan with a fan drive signal which causes the at least one fan to operate with a fan speed,
wherein the controller is further adapted to process a fan speed signal thereby to provide an indication of a level of leakage to or from the mask volume.

This face mask determines a level of leakage of the mask, so that a wearer may be instructed to make adjustments to the mask. The leakage detection is based on information about a sensed fan speed. The sensed fan speed may be obtained from an existing signal provided by the fan. Thus, the leakage detection may be performed with existing sensors which are already present for other purposes. The fan speed may instead be sensed with a dedicated sensor. By way of example, sensing of the fan speed may be achieved by using fan current detection, a hall sensor, or an optical sensor.

The leakage level is fed back to the wearer by the indication provided. By reducing leakage, the protection provided by the mask is improved. The indication of a leakage level may be a simple binary indicator (yes/no) or it may be a multi-level signal indicating different degrees of mask leakage. The indication may provide advice as to whether or not mask adjustment is needed.

The controller is for example adapted to obtain a fan speed signal during a testing phase during which the wearer is instructed to hold their breath.

Thus, the wearer simply needs to hold their breath for a short time (typically a time period between 5 second and 20 seconds) so that the mask conditions can be determined without the complicating influence of the wearer's breath.

The controller is for example adapted to compare the speed signal with reference data which relates to a no leakage condition. Thus, a deviation from fan speed conditions which relate to a no leakage condition is detected and used as an indicator of mask leakage.

There may be a memory which stores the reference data as fan specification information. This may for example be in the form of a function between the flow rate and the pressure difference across the fan for a particular (e.g. maximum) fan drive signal. The flow rate is proportional to the fan speed so that sensing of the fan speed maybe considered equivalent to sensing of the flow rate.

The controller may instead be adapted to obtain the reference data during a calibration phase during which the wearer is instructed to hold their breath and to hold the mask to manually prevent leakage from the mask volume, and wherein the face mask further comprises a memory which stores the reference data. Thus, the reference data may be obtained by physically ensuring there is no leakage while performing a test.

As a further alternative, for determining the reference data, the mask (or indeed a mask of the same design) could be tested during a factory calibration phase to obtain the reference data. This could involve applying the mask to a specially designed template for which it is known that no leakage arises (because the template is designed for the mask, whereas a user's face is not).

The controller may be adapted to determine the breathing pattern of the wearer and to control the fan arrangement in dependence on the breathing pattern. This sensed pressure difference may be obtained by a dedicated sensor, or it may be derived from the fan speed sensor signal, which itself will fluctuate with the breathing cycle.

The controller may be adapted to control the fan arrangement to assist inhalation of the wearer. In such a case, it provides a positive pressure difference to the mask volume.

The controller may instead be adapted to control the fan arrangement to assist exhalation of the wearer. In such a case, it provides a negative pressure difference to the mask volume.

The fan arrangement may instead comprise different fans for assisting exhalation and inhalation respectively, or a single fan operable in opposite flow directions for assisting inhalation and exhalation sequentially. The pressure inside the mask volume will then depend on the fan in use or the fan flow direction being applied.

The face mask may further comprise a pressure sensor arrangement for sensing a pressure difference between the mask volume and the ambient surroundings outside the mask volume. This pressure difference signal may be used in combination with the fan speed signal to derive the leakage level.

The differential pressure is often obtained for the purposes of detecting the breathing cycle of the wearer so that the fan arrangement may be controlled in synchronism with the breathing of the wearer. Thus, detecting differential pressure may also be achieved with existing sensors.

The invention also provides a method of operating a fan-assisted face mask comprising a mask body which is adapted to enclose a mask volume against the face of a wearer and a fan arrangement comprising at least one fan, the method comprising the steps of:

controlling the at least one fan with a fan drive signal which causes the at least one fan to operate with a fan speed; and
processing a fan speed signal thereby to provide an indication of a level of leakage to or from the mask volume.

This method is used by the face mask described above, to provide the indication of leakage.

The method may comprise obtaining the fan speed signal during a testing phase during which the wearer is instructed to hold their breath.

The method may comprise comparing the fan speed signal with reference data which relates to a no leakage condition. The reference data may be stored as fan specification information, or else it may be obtained and stored during a calibration phase during which the wearer is instructed to hold their breath and to hold the mask to manually prevent leakage from the mask volume.

The method may comprises determining the breathing pattern of the wearer and controlling the fan arrangement in dependence on the breathing pattern.

The invention also provides a computer program comprising computer program code means which is adapted, when said computer program is run on a processor in a face mask, to implement the method described above. Thus, the invention may be implemented at least in part in software.

These and other aspects of the invention will be apparent from and elucidated with reference to the embodiments described hereinafter.

BRIEF DESCRIPTION OF THE DRAWINGS

Examples of the invention will now be described in detail with reference to the accompanying drawings, in which:

Figure 1 is a sectional side view of a mask in accordance with the invention, fitted on the face of a wearer;
Figure 2 is a graph showing the variation of pressure difference with air flow rate of a typical blower fan that may be used in the mask of Figure 1;
Figure 3 shows the relationship between flow rate and pressure difference for the fan, operating at a particular fan setting.
Figure 4A shows a plot of flow rate over time and Figure 4B shows the pressure difference for a condition with no leakage;
Figure 5A shows a plot of flow rate over time and Figure 5B shows the pressure difference when a small leak is introduced;
Figure 6A shows a plot of flow rate over time and Figure 6B shows the pressure difference when a large leak is introduced; and
Figure 7 shows a method of operating a fan-assisted face mask to provide an indication of a level of leakage to or from the mask volume.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a fan-assisted face mask in which a fan speed sensing signal is processed, to provide an indication of a level of leakage to or from the mask volume. In this way, a wearer of the mask is able to ensure correct initial fitting of the mask, as well as to perform periodic checks of the fitting of the mask. Optionally, there is also a pressure sensor arrangement for sensing a pressure difference between a mask volume and the ambient surroundings outside the mask volume. The fan speed signal and the sensed pressure difference may then be processed together to provide the indication of a level of leakage. Pressure sensing is optional, but if there is already pressure sensing as part of the mask design, the use of pressure information in addition to fan speed information may enable more accurate results to be achieved.

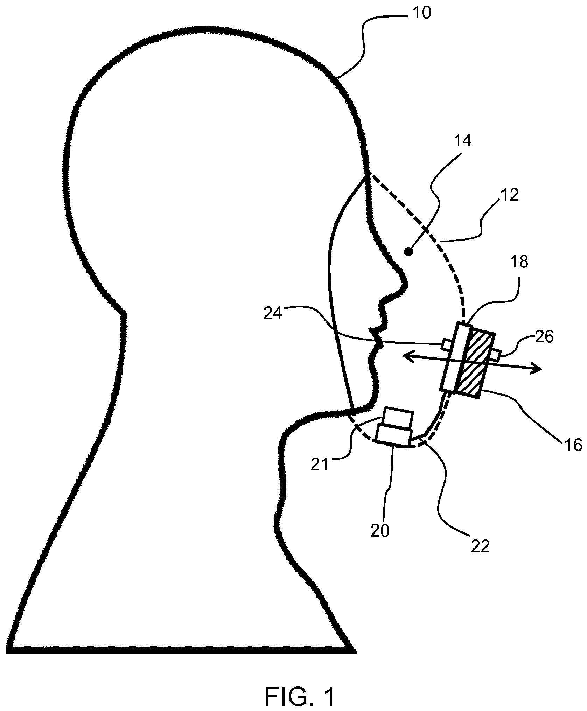

Figure 1 shows a subject 10 wearing a face mask 12 which covers the nose and mouth of the wearer. The purpose of the mask is to filter ambient air before it is breathed in by the wearer and to provide active control of the flow of air into an air chamber 14, i.e. the mask volume. A fan 16 draws in ambient air through a filter 18 which is in series with the fan.

A part of the remainder of the mask area is formed of an air permeable filter so that the user may breathe in and out through the mask. Thus air is drawn into the air chamber 14 by lung inhalation through the mask wall and with assistance provided by the fan acting as an air pump.

Figure 1 is only a schematic representation of one general example. Typically, there is also a check valve, which opens during exhaling and closes during inhaling. The fan can be on top of the check valve if the fan is used to blow the air out. In such a case, the filter 18 is not needed, and the mask body itself performs the filtering of air to be inhaled..

The controller, memory, fan and sensor are shown as separate units in separate locations, but of course they maybe formed together as a single module.

In one example, the fan motor is an electronically commutated brushless motor. Such motors have internal sensor which enables the motor speed and hence fan speed to be measured. In particular, electronically commutated brushless DC fans have internal sensors that measure the position of the rotor and switch the current through the coils in such a way that the rotor rotates. An internal sensor is thus already provided in such motors to enable determination of the motor and hence fan speed.

During exhalation, air is expelled from the air chamber 14. In this example, exhalation is through the same air pump arrangement and hence also through the filter 18, as well as through the air permeable part of the mask body, even though the expelled air does not need to be filtered.

As a further option, a one-way check valve (not shown in Figure 1) in the mask wall may allow exhaled air to be expelled. Since the mask chamber 14 is closed by the face of the user, the pressure inside the closed chamber when the mask is worn will vary as a function of the breathing cycle of the subject. When the subject breathes out, there will be a slight pressure increase and when the subject breathes in there will be a slight pressure reduction. A check valve may thus avoid the need for the wearer to breathe out through the mask wall.

In some examples, the operation of the fan arrangement is also timed with the breathing cycle of the user. The breathing cycle is identified by a controller 20, which for example receives a differential pressure signal from a pressure sensor arrangement 24, 26 by a wired connection 22. The controller 20 also controls the operation of the fan. The breathing cycle timing is used by the controller to time the periods of operation of the fan. The controller has access to an associated memory 21.

The pressure sensor is optional and is not needed to perform the leakage detection of the invention. If a pressure sensor is present, its signal may however also be processed in the manner explained below.

Furthermore, the breathing cycle timing may be obtained from a fan speed signal (instead of based on pressure sensing), since for a given fan drive level, fluctuations in fan speed will be observed which correlate with the breathing of the wearer. Thus, the fan speed signal may also be used as a proxy for the differential pressure, and the fan speed signal may be used to control the fans as a function of the breathing cycle (by detecting the end of inhalation and then switching to exhalation control, and detecting the end of exhalation and then switching to inhalation control).

In the example of Figure 1, a pressure sensor arrangement is shown as a first pressure sensor 24 inside the mask volume and a 26 outside the mask volume. A single sensor may instead be provided, which is integrated into the mask wall and is exposed to both sides. Alternatively, only a pressure sensor inside the mask volume may be needed. The ambient pressure may be obtained from a remote pressure sensor for example of a different piece of equipment or from an external database, since it is simply the prevailing ambient pressure.

In examples where there is only one fan 16, the fan may be controlled to operate only to expel air, to assist exhalation, or it may be controlled to operate only to intake air, to assist inhalation. Alternatively, it may be operable bi-directionally, i.e. in opposite directions of flow, to assist sequentially in exhalation and inhalation of air through the filter 18.

In other examples, there may be two fans, operable to assist respectively in the exhalation and inhalation of air through the filter or through respective filters. They may be timed by the same controller 20.

A power source such as a battery (not shown), preferably rechargeable, is arranged in the mask to power the control unit 20, pressure sensor arrangement 26 and the fan 16.

The controller 20 provides a fan drive signal which causes the fan to operate with a fan speed. The resulting fan speed is then sensed. In the case of an electrically commutated brushless DC fan, a signal is available which indicates the fan speed, and this may be termed a "fan speed signal". A separate sensor may instead be used. In all cases, there is a "fan speed signal" which is indicative of the fan speed. Such a fan speed signal is then indicative of the flow rate through the fan. It may also be used as an indicator of the pressure difference between the mask cavity and the ambient surroundings, thus avoiding the need for a separate pressure sensor.

The controller processes (at least) the fan speed signal (which is related to the pressure difference), to provide an indication of a level of leakage to or from the mask volume. The way this can be achieved is described below.

By determining a level of leakage of the mask, a wearer may be instructed to make adjustments to the mask. By reducing leakage with suitable mask adjustments, the protection provided by the mask is improved. The indication of a leakage level may be a simple binary indicator (yes/no) or it may be a multi-level signal indicating different degrees of mask leakage. The indication may provide advice as to whether or not mask adjustment is needed.

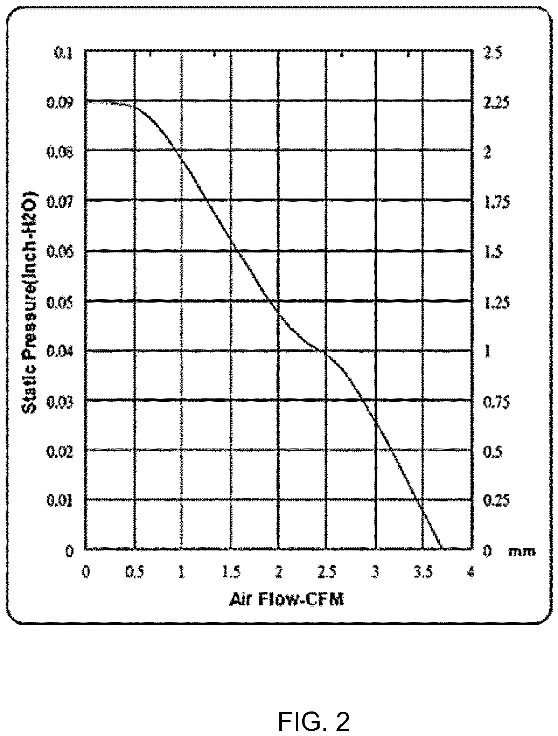

The factory specifications of the fan 16 are predetermined, and in particular the variation of flow velocity and pressure difference is shown for a typical blower fan in Figure 2 when operated at a particular (e.g. maximum) drive level. Static pressure is shown on the vertical scale in inch-H2O on the left and in cm-H2O on the right. Flow rate is shown on the horizontal scale in cubic feet per minute (there are about 1076 cubic feet per cubic meter). The static pressure is the pressure on one side of the fan relative to the pressure at the other side, and thus is a measure of pressure difference.

A fan working at a particular pressure difference thus generates a corresponding airflow. Once the user wears the mask and the fan starts rotation, the fan will generate a certain pressure inside the mask. The invention is based obtaining an indication of the fan rotation speed (and hence air flow rate).

Leakage is caused by a the presence of a slit between the face and the mask. The leakage will cause particulate matter to enter the mask volume and reduce the mask protection level, especially during inhalation. The leakage will also cause a change in the flow rate and the pressure difference. The flow resistance introduced by the slit will depend on the dimensions of the slit and the viscosity of air. The flow resistance depends on the dynamic viscosity and the slit dimensions (the height and width of the slit i.e. the slit opening dimensions and the depth of the slit).

For a given pressure, the air flow rate Q is proportional to the fan rotation speed, i.e.:

$$Q_1/Q_0 = n_1/n_0$$

where $Q_1$ and $Q_0$ are flow rates at the different fan rotation speeds $n_1$ and no. A higher fan rotation speed is needed to create an increased air flow.

The flow rate is a function of the pressure difference:

$$Q=f(P)$$

Hence:

$$P=f^{-1}(Q)$$

The relationship $Q=f(P)$ may be obtained from the fan specification data such as shown in Figure 2. The function may be modeled as a look up table, or with regression modeling of data the function can be known in algebraic terms. It enables a mapping between fan speed and pressure, so that fan speed monitoring is able to be used to detect the breathing cycles of the wearer.

By way of example, if the mask is fitted well, then $Q_0$, $P_0$, $n_0$ may be default values, whereas $Q_1$, $P_1$, $n_1$ are values which arise when there is some leakage. Leaking will make $Q_1$ increase, so that $n_1$ will also increase.

Thus, changes in fan rotation speed beyond those expected (in a no leakage case) for the observed pressure can be used as an indicator of leakage. As discussed below, by performing the assessment when the wearer holds their breath, the effect of breathing on the pressure in the mask is cancelled, and the monitoring of fan speed provides an accurate analysis of the steady state condition of the mask. Thresholds for the fan rotation speed may be set to indicate the leakage level:

$n_1 - n_0 < T_{low}$, indicates small leakage which is acceptable;

$T_{low} < n_1 - n_0 < T_{high}$ indicates some leakage, and the mask has to be adjusted.

$n_1 - n_0 > T_{high}$ indicates excessive leakage.

The threshold values $T_{low}$ and $T_{high}$ can be defined for different types of fan. The fan speed alone can then be used as a measure of leakage.

Note that, equivalently, changes in the pressure value may be used as an indication of the departure from the no leak condition, with thresholds set for the pressure difference which is achieved for a given fan drive signal.

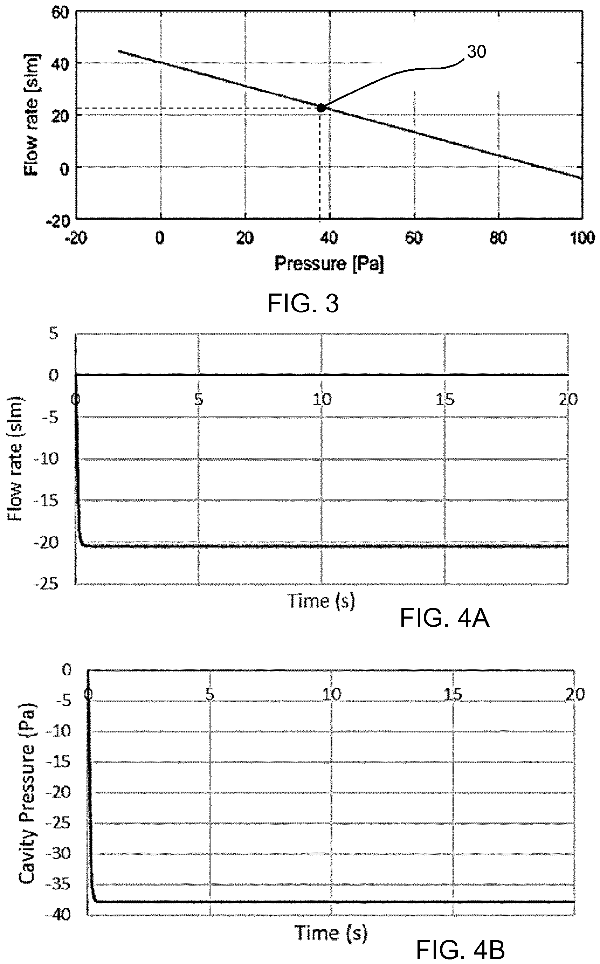

The method has been simulated and the results are shown in Figures 3 to 6. The simulation was performed for an outlet fan, which hence results in a pressure drop in the mask volume, i.e. a negative pressure difference relative to the ambient pressure.

Figure 3 shows the relationship between flow rate and pressure difference for the fan, operating at a particular fan setting. It shows that there is a direct relationship between flow rate (which depends on fan speed) and pressure, so that flow rate measurement (i.e. fan speed measurement) may be used as a measure of pressure, for example to identify breathing cycles of the wearer.

The flow measurements (and optionally also pressure measurements, if actual pressure measurement is also being used) are taken during a test period (for example of up to 20 seconds) when the wearer does not breathe.

Figure 4A shows a plot of flow rate over time (in standard liters per minute) and Figure 4B shows the pressure difference. These plots relate to a condition with no leakage.

The pressure difference and flow rate is constant after an initial settling period, because the mask reaches a steady state, as a result of the flow through the mask wall.

The flow rate of -21 slm and the cavity pressure of -38 Pa are shown as the operating point 30 on the fan characteristic function of Figure 3.

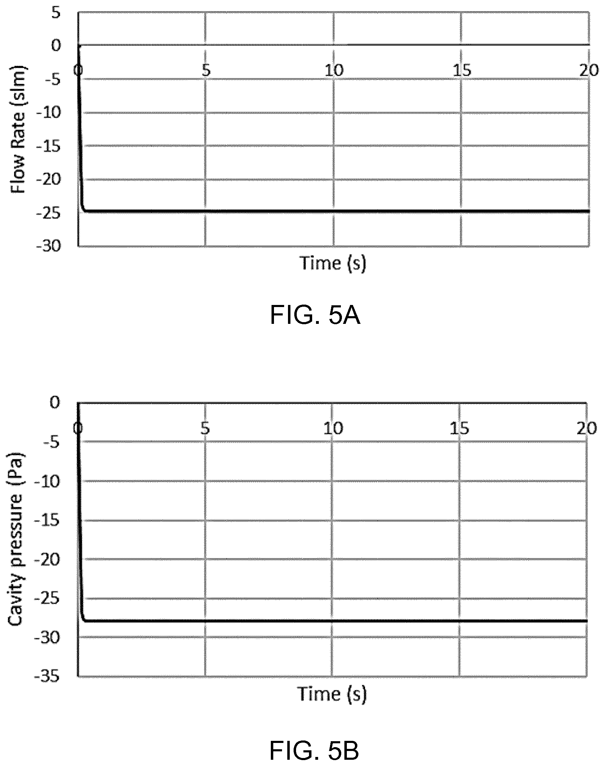

Figure 5A shows a plot of flow rate over time (in standard liters per minute) and Figure 5B shows the pressure difference when a small leak is introduced into the model. The small leak is modelled as a slit with crevice width 200mm, crevice height 300$\mu$m, and crevice length 10mm.

The flow rate has increased in magnitude to -25 slm and the pressure difference has dropped in magnitude to -28 Pa.

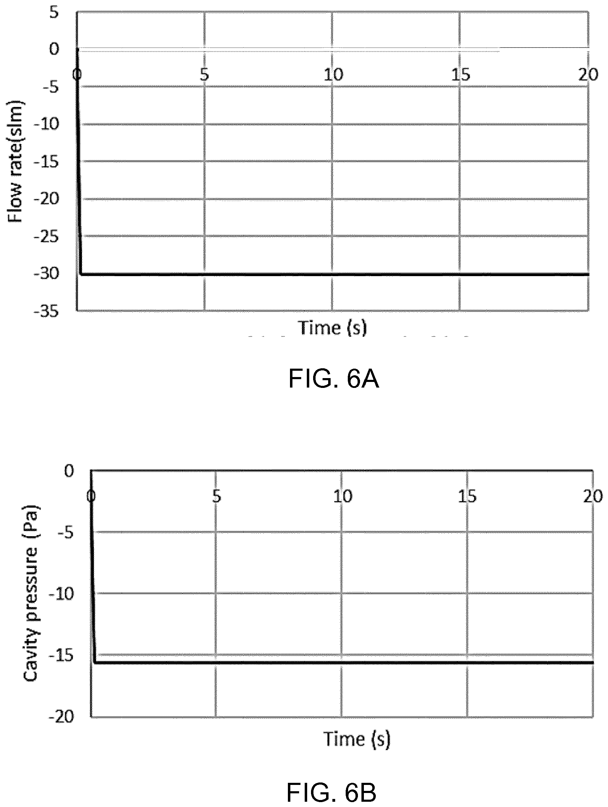

Figure 6A shows a plot of flow rate over time (in standard liters per minute) and Figure 6B shows the pressure difference when a large leak is introduced into the model. The large leak is modeled as a slit with crevice width 200mm, crevice height 500$\mu$m, and crevice length 20mm. The flow rate has increased in magnitude to -30 slm and the pressure difference has dropped in magnitude to -16 Pa.

These results clearly show that both the pressure and the flow rate differ according to the leakage level. As a result, according to the pressure difference or the flow rate compared to the default set of values, the user can be advised whether they are wearing the mask properly or not.

The leakage test is typically performed when the mask is first fitted, but it may be performed periodically while the mask is in use.

The user puts on the mask, adjusts the straps and turns on the fan. The user may then input a test command (e.g. by pressing a test button) after which the user holds their breath for a certain time. This provides a testing phase. A period of a few seconds is adequate, for example up to 20s. The time may be configurable by the user, according to how comfortable they are holding their breath. When the time period has expired, the controller performs analysis of the speed data during the breath holding.

If the leakage is found to be acceptable, the user can use the mask normally. Otherwise, a warning is provided such as as LED indication, buzzer sound, or a warming message on a smart phone, such as "please adjust the mask strap". The user then adjusts the mask and conducts a further test.

As mentioned above, the mask leakage is determined with reference to conditions for a non-leaking mask, which default set of values will be termed "reference data". The reference data may be stored in memory as fan specification information, for example data of the type shown in Figure 2.

Instead, the reference data may be generated during a calibration. The wearer may again be instructed to hold their breath but also to hold the mask to manually prevent leakage from the mask volume. Thus, the reference data may in this case be obtained by physically ensuring there is no leakage while performing a test rather than relying on more theoretical fan characteristics data.

As a further alternative, the mask type may be subjected to a factory calibration phase to obtain the reference data. This could involve ensuring a no leakage condition, for example applying the mask to a specially designed no leakage template.

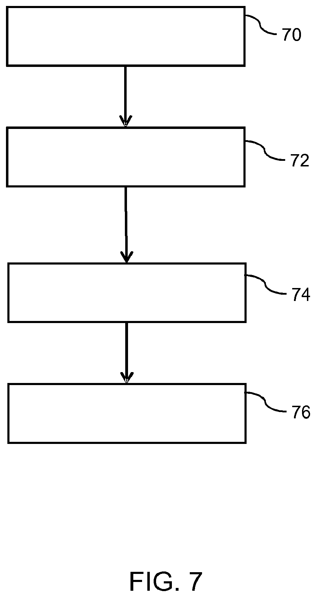

Figure 7 shows a method of operating an electric fan-assisted face mask comprising a mask body which is adapted to enclose a mask volume against the face of a wearer and a fan arrangement comprising at least one fan. The method comprises:

optional step 70, of sensing a pressure difference between the mask volume and the ambient surroundings outside the mask volume;
in step 72, controlling the at least one fan with a fan drive signal which causes the at least one fan to operate with a fan speed; and
in step 74, processing a fan speed sensing signal (and a pressure difference signal if one has been obtained separately from the fan speed signal) thereby to provide an indication of a level of leakage to or from the mask volume.

The method is carried out during a testing phase during which the wearer is instructed to hold their breath.

As explained above, the processing makes use of reference data which relates to a no leakage condition.

After the testing, the method comprises in step 76 determining the breathing pattern of the wearer based on the sensed pressure difference and controlling the fan arrangement in dependence on the breathing pattern. Thus, the pressure difference sensing has multiple purposes and is part of the normal operation of the mask after the testing phase.

As discussed above, embodiments make use of a controller. The controller can be implemented in numerous ways, with software and/or hardware, to perform the various functions required. A processor is typically used, which employs one or more microprocessors that maybe programmed using software (e.g., microcode) to perform the required functions. The controller may however be implemented with or without employing a processor, and also may be implemented as a combination of dedicated hardware to perform some functions and a processor (e.g., one or more programmed microprocessors and associated circuitry) to perform other functions.

Examples of control components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media maybe fixed within a processor or controller or may be transportable, such that the one or more programs stored thereon can be loaded into a processor or controller.

The mask may include wireless data transmission capability (e.g. Wifi or Bluetooth), for example for communicating information to a portable device of the wearer such as a mobile phone or tablet.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

**1.** A fan-assisted face mask comprising:

a mask body (12) which is adapted to enclose a mask volume (14) against the face of a wearer (10);

a fan arrangement (16) comprising at least one fan; and

a controller (20) adapted to control the at least one fan with a fan drive signal which causes the at least one fan to operate with a fan speed, wherein the controller (20) is further adapted to process a fan speed signal thereby to provide an indication of a level of leakage to or from the mask volume.

2. A face mask as claimed in claim 1, wherein the controller (20) is adapted to:

obtain the fan speed signal during a testing phase during which the wearer is instructed to hold their breath.

3. A face mask as claimed in claim 1 or 2, wherein the controller (20) is adapted to:

compare the fan speed signal with reference data which relates to a no leakage condition.

4. A face mask as claimed in claim 3, comprising a memory (21) which stores the reference data as fan specification information.

5. A face mask as claimed in claim 3, wherein the controller (20) is adapted to obtain the reference data during a calibration phase during which the wearer is instructed to hold their breath and to hold the mask to manually prevent leakage from the mask volume, and wherein the face mask further comprises a memory (21) which stores the reference data.

6. A face mask as claimed in any preceding claim, wherein the controller (20) is adapted to determine the breathing pattern of the wearer and to control the fan arrangement in dependence on the breathing pattern.

7. A face mask according to any preceding claim, wherein the controller (20) is adapted to control the fan arrangement to assist inhalation of the wearer or to control the fan arrangement to assist exhalation of the wearer.

8. A face mask according to any preceding claim, wherein the fan arrangement (16) comprises different fans for assisting exhalation and inhalation respectively, or a single fan operable in opposite flow directions for assisting inhalation and exhalation sequentially.

9. A face mask according to any preceding claim, further comprising a pressure sensor arrangement (24, 26) for sensing a pressure difference between the mask volume (14) and the ambient surroundings outside the mask volume.

10. A method of operating a fan-assisted face mask comprising a mask body which is adapted to enclose a mask volume against the face of a wearer and a fan arrangement comprising at least one fan, the method comprising the steps of:

(72) controlling the at least one fan with a fan drive signal which causes the at least one fan to operate with a fan speed; and

(74) processing a fan speed signal, thereby to provide an indication of a level of leakage to or from the mask volume.

11. A method as claimed in claim 10 comprising obtaining the fan speed signal during a testing phase during which the wearer is instructed to hold their breath.

12. A method as claimed in claim 10 or 11, comprising comparing the fan speed signal with reference data which relates to a no leakage condition.

13. A method as claimed in claim 12, comprising:

storing the reference data as fan specification information; or

obtaining and storing the reference data during a calibration phase during which the wearer is instructed to hold their breath and to hold the mask to manually prevent leakage from the mask volume.

14. A method as claimed in any one of claims 10 to 13 comprising (76) determining the breathing pattern of the wearer and controlling the fan arrangement in dependence on the breathing pattern.

15. A computer program comprising computer program code means which is adapted, when said computer program is run on a processor in a face mask, to implement the method of any one of claims 10 to 14.

FIG. 1

FIG. 2

FIG. 3

FIG. 4A

FIG. 4B

FIG. 5A

FIG. 5B

FIG. 6A

FIG. 6B

FIG. 7

Europäisches
Patentamt

European
Patent Office

Office européen
des brevets

# EUROPEAN SEARCH REPORT

Application Number

EP 18 17 4893

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GB 2 472 592 A (3M INNOVATIVE PROPERTIES CO [US]) 16 February 2011 (2011-02-16) * page 6, line 14 - page 11, line 25 * * figures 1-4 * | 1-15 | INV. A62B18/08 A61M16/06 A62B9/00 G01M3/26 |
| Y | US 2013/333702 A1 (BALOA WELZIEN LEONARDO ALBERTO [US] ET AL) 19 December 2013 (2013-12-19) * paragraph [0053] * * figures * | 1-15 | |
| A | GB 2 032 284 A (RACAL SAFETY LTD) 8 May 1980 (1980-05-08) * page 2, line 24 - page 3, line 123 * * figures 2,3,6,7 * | 1-15 | |

TECHNICAL FIELDS
SEARCHED      (IPC)

A62B
A61M
G01M

The present search report has been drawn up for all claims

| Place of search | Date of completion of the search | Examiner |
|---|---|---|
| The Hague | 9 November 2018 | Nehrdich, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 18 17 4893

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2018

| Patent document cited in search report | | Publication date | Patent family member(s) | | Publication date |
|---|---|---|---|---|---|
| GB 2472592 | A | 16-02-2011 | AU | 2010282612 A1 | 23-02-2012 |
| | | | CN | 102470260 A | 23-05-2012 |
| | | | EP | 2464429 A1 | 20-06-2012 |
| | | | GB | 2472592 A | 16-02-2011 |
| | | | JP | 5723366 B2 | 27-05-2015 |
| | | | JP | 2013501585 A | 17-01-2013 |
| | | | KR | 20120051735 A | 22-05-2012 |
| | | | US | 2012138051 A1 | 07-06-2012 |
| | | | WO | 2011019778 A1 | 17-02-2011 |
| US 2013333702 | A1 | 19-12-2013 | EP | 2861139 A1 | 22-04-2015 |
| | | | US | 2013333702 A1 | 19-12-2013 |
| | | | WO | 2013188013 A1 | 19-12-2013 |
| GB 2032284 | A | 08-05-1980 | NONE | | |

EPO FORM P0459

For more details about this annex : see Official Journal of the European Patent Office, No. 12/82